# EUROPEAN PATENT APPLICATION

(11) **EP 4 563 096 A2**
(43) Date of publication of application: **04.06.2025**
(21) Application number: 25164114.8
(22) Date of filing: 15.10.2020
(51) Int. Cl.: A61B 17/04

(54) **FILAMENT CUTTING DEVICES**

(30) Priority: 18.10.2019 US 201962923042 P; 17.04.2020 US 202063011388 P
(62) Divisional of application: 20804047.7
(71) Applicant: Boston Scientific Scimed, Inc., Maple Grove, MN 55311 (US)
(72) Inventor: The designation of the inventor has not yet been filed
(74) Representative: Vossius & Partner Patentanwälte Rechtsanwälte mbB

(57) **Abstract**

The present invention discloses a filament cutting device, comprising: an outer sheath; a bushing coupled to a distal end of the outer sheath, wherein the bushing includes a cutting edge; an actuation wire slidably extendable within the outer sheath and bushing; and an engaging body coupled to a distal end of the actuation wire, the engaging body comprising a cavity defined along a length of the engaging body configured to capture a portion of a filament within the cavity, the engaging body including at its distal portion a sloped surface slidable along the filament as the engaging body are translated distally into contact with the filament; and wherein movement of the actuation wire and engaging body with the filament captured within the cavity causes the cutting edge to sever the filament.

## Description

### CROSS-REFERENCE TO RELATED APPLICATIONS

This application claims the benefit of priority under 35 U.S.C. § 119 to U.S. Provisional Application No. 63/011,388, filed April 17, 2020, and U.S. Provisional Application No. 62/923,042, filed October 18, 2019, both of which disclosures are incorporated herein by reference in their entireties for all purposes.

### FIELD

This disclosure relates generally to the field of devices and procedures for severing a filament, and particularly in use for severing filaments of tether devices as part of tether traction systems and procedures.

### BACKGROUND

Access to and severing of one or more filaments during a medical procedure, *e.g.,* a filament in use as a tether in a tissue dissection procedure, may be difficult to perform by a medical professional because of remote access to the filament, visualization, tortious anatomies, establishing sufficient shear force, or the like.

A variety of advantageous medical outcomes may be realized by the embodiments of the present disclosure.

### SUMMARY

Various embodiments of filament cutting devices, systems, and methods are described herein. For example, use of filament cutting devices in systems including tether devices having filaments, which may be delivered into a body lumen of a patient and deployed to apply traction to tissue during a dissection procedure, *e*.*g*., endoscopic mucosal resection and/or endoscopic submucosal dissection (EMR/ESD), and which may be retrieved after the procedure by severing the filament with such filament cutting devices, are described. Exemplary tether devices and/or tether delivery devices for use together, alone, and/or in combination with filaments cutting devices, in such systems or other systems, are also described herein.

In an aspect, a filament cutting device may include an outer sheath. A bushing may be coupled to a distal end of the outer sheath. An inner diameter of the bushing may include a cutting edge. An actuation wire may be slidably extendable within the outer sheath and bushing. An engaging body may be coupled to a distal end of the actuation wire. The engaging body may include an outer surface having a diameter that substantially matches an inner diameter of the cutting edge of the bushing. A cavity may be defined along a length of the engaging body configured to capture a portion of the filament within the cavity. Movement of the actuation wire and engaging body with the filament captured within the cavity may cause the cutting edge to sever the filament.

In various of the described and other aspects, a proximal portion of the cavity may include an angled sloping surface. A distal portion of the cavity may include an innermost curvature of the cavity defining a hook shape. The innermost curvature of the cavity may extend radially within the engaging body a length greater than 50% of a diameter of the engaging body. The cutting edge may be at a distal tip of the bushing. A cutting cavity may be defined along a length of the bushing, and the cutting edge may be along the cutting cavity. The outer sheath may comprise winding coils, and a distal tip of the outer sheath may comprise a ground outer surface where the bushing is coupled to the outer sheath. A proximal portion of the outer sheath may include a smaller inner diameter than a remainder of the outer sheath, and the proximal portion of the outer sheath may include a smaller outer diameter than the remainder of the outer sheath. The engaging body may include a second cavity substantially opposing the first cavity about a longitudinal axis of the engaging body. The engaging body may include a substantially square outer perimeter that substantially matches an inner perimeter of the bushing.

In an aspect, a filament cutting device may include an outer sheath. A bushing may be coupled to a distal end of the outer sheath. A cavity may be defined along a length of the bushing and may be configured to capture a portion of a filament within the cavity. An actuation wire may be slidably extendable within the outer sheath and the bushing. An engaging body may be coupled to a distal end of the actuation wire. The engaging body may include a cutting edge at a distal tip of the engaging body. Movement of the actuation wire and engaging body with the filament captured within the cavity may cause the cutting edge to sever the filament.

In various of the described and other aspects, the cutting edge may be an outer diameter of the engaging body. A distal tip of the engaging body may include a surface having an angle extending from a longitudinal axis of the engaging body to the cutting edge. A contact body may be disposed within a distal end of the bushing configured to prevent distal translation of the engaging body. The contact body may include a tapered proximal portion that tapers proximally with a decreasing width. The engaging body may include a tapered distal portion that tapers distally with a decreasing width.

In an aspect, a filament cutting device may include an outer sheath. A bushing may be coupled to a distal end of the outer sheath. The bushing may include a cavity. A cutter may extend across the cavity and may be configured to sever the filament. The cavity may be defined along a length of the bushing and may be configured to capture a portion of a filament within the cavity. The cutter may be a blade having an edge extending substantially parallel with a longitudinal axis of the filament cutting device. The cavity may be defined transversely across a distal tip of the bushing. The cutter may be an activatable wire configured to melt the filament.

In an aspect, a filament cutting device may include an outer sheath. A bushing may be coupled to a distal end of the outer sheath. An inner diameter of the bushing may be a cutting edge at a distal tip of the bushing. An actuation wire may be slidably extendable within the outer sheath and bushing. An engaging body may be coupled to a distal end of the actuation wire. The engaging body may include an outer surface having a diameter that substantially matches an inner diameter of the cutting edge of the bushing. A cavity may be defined along a length of the engaging body and may be configured to capture a portion of the filament within the cavity. Movement of the actuation wire and engaging body with the filament captured within the cavity may cause the cutting edge to sever the filament. The filament may be positionable at least partially in the cavity of the engaging body such that in response to proximal movement of the engaging body into the bushing, the filament is severable via the bushing and/or an edge of the cavity.

In another aspect, a system may include a filament cutting device, such as the filament cutting devices described above and elsewhere herein. The system may include a tether device. The system may include a tether delivery device.

In an aspect, a tether device may include a tether having a distal end, a proximal end, and a stretchable elongate body extending therebetween. The proximal end of the tether may be configured to be attached to a deployable clipping device at a distal end of a delivery catheter. The distal end of the tether may be configured with a loop extending from a neck. The loop may be configured to be engaged by a second deployable clipping device at the distal end of a delivery catheter. The loop and neck may comprise a filament that may be severable by a filament cutting device, such as the filament cutting devices described above and elsewhere herein.

In another aspect, a method may include extending an engaging body of a filament cutting device toward a filament. The filament may be captured within a cavity of the engaging body. The engaging body may be retracted into an outer sheath, thereby severing the filament.

### BRIEF DESCRIPTION OF THE DRAWINGS

Non-limiting embodiments of the present disclosure are described by way of example with reference to the accompanying figures, which are schematic and not intended to be drawn to scale. In the figures, each identical or nearly identical component illustrated is typically represented by a single numeral. For purposes of clarity, not every component is labeled in every figure, nor is every component of each embodiment shown where illustration is not necessary to allow those of ordinary skill in the art to understand the disclosure. In the figures:
FIG. 1 illustrates a filament cutting device, according to an embodiment of the present disclosure.
FIG. 2 illustrates an actuation element and an inner sheath of a filament cutting device, according to an embodiment of the present disclosure.
FIG. 3A illustrates longitudinal translation of a filament cutting device, according to an embodiment of the present disclosure.
FIG. 3B illustrates rotation of the filament cutting device of FIG. 3A.
FIG. 3C illustrates a cross-sectional view of a proximal end of the device of FIGS. 3A and 3B.
FIG. 3D illustrates a cross-sectional perspective view of the proximal end of the device of FIGS. 3A-3C.
FIG. 4A illustrates a proximal portion of an assembled filament cutting device, according to an embodiment of the present disclosure.
FIG. 4B illustrates the device of FIG. 4A partially unassembled.
FIGS. 5A-5D illustrate a distal end of a filament cutting device being translated longitudinally and engaging and severing a filament, according to an embodiment of the present disclosure.
FIG. 6 illustrates a profile of a cavity of an engaging body of a filament cutting device, according to an embodiment of the present disclosure.
FIG. 7 illustrates a cross-sectional view of a bushing having an edge, according to an embodiment of the present disclosure.
FIG. 8 illustrates an outer sheath of a filament cutting device, according to an embodiment of the present disclosure.
FIG. 9 illustrates an engaging body of a filament cutting device, according to an embodiment of the present disclosure.
FIGS. 10A-10D illustrate a distal end of a filament cutting device being translated longitudinally and engaging and severing a filament, according to an embodiment of the present disclosure.
FIGS. 11A and 11B illustrate an engaging body and a bushing, according to an embodiment of the present disclosure.
FIG. 12 illustrates an engaging body, according to an embodiment of the present disclosure.
FIGS. 13A-13D illustrate an engaging body in various positions with respect to a bushing for engaging and severing a filament, according to an embodiment of the present disclosure.
FIG. 14 illustrates an engaging body and a bushing, according to an embodiment of the present disclosure.
FIG. 15 illustrates an engaging body and a bushing, according to an embodiment of the present disclosure.
FIGS. 16A-16C illustrate an engaging body in various positions with respect to a bushing for engaging and severing a filament, according to an embodiment of the present disclosure.
FIG. 17 illustrates a bushing having a cutter that is a blade, according to an embodiment of the present disclosure.
FIG. 18 illustrates a bushing having a cutter that is an activatable wire, according to an embodiment of the present disclosure.
FIG. 19 illustrates a tether device and a clip delivery device, according to an embodiment of the present disclosure.
FIG. 20 illustrates a tether device deployed in a body lumen, according to an embodiment of the present disclosure

### DETAILED DESCRIPTION

The present disclosure is not limited to the particular embodiments described herein. The terminology used herein is for the purpose of describing particular embodiments only and is not intended to be limiting beyond the scope of the appended claims. Unless otherwise defined, all technical terms used herein have the same meaning as commonly understood by one of ordinary skill in the art to which the disclosure belongs.

As used herein, the singular forms "a," "an," and "the" are intended to include the plural forms as well, unless the context clearly indicates otherwise. The terms "comprises" and/or "comprising," or "includes" and/or "including" when used herein, specify the presence of stated features, regions, steps elements and/or components, but do not preclude the presence or addition of one or more other features, regions, integers, steps, operations, elements, components and/or groups thereof. As used herein, the conjunction "and" includes each of the structures, components, features, or the like, which are so conjoined, unless the context clearly indicates otherwise, and the conjunction "or" includes one or the others of the structures, components, features, or the like, which are so conjoined, singly and in any combination and number, unless the context clearly indicates otherwise. The term "or" is generally employed in its sense including "and/or" unless the content clearly dictates otherwise.

Various embodiments according to the present disclosure are described below. As used herein, "proximal end" refers to the end of a device that lies closest to the medical professional along the device when introducing the device into a patient, and "distal end" refers to the end of a device or object that lies furthest from the medical professional along the device during implantation, positioning, or delivery.

All numeric values are herein assumed to be modified by the term "about," whether or not explicitly indicated. The term "about", in the context of numeric values, generally refers to a range of numbers that one of skill in the art would consider equivalent to the recited value (*i.e.,* having the same function or result). In many instances, the term "about" may include numbers that are rounded to the nearest significant figure. Other uses of the term "about" (*i.e.,* in a context other than numeric values) may be assumed to have their ordinary and customary definition(s), as understood from and consistent with the context of the specification, unless otherwise specified. The recitation of numerical ranges by endpoints includes all numbers within that range, including the endpoints (*e*.*g*. 1 to 5 includes 1, 1.5, 2, 2.75, 3, 3.80, 4, and 5).

It is noted that references in the specification to "an embodiment", "some embodiments", "other embodiments", *etc.,* indicate that the embodiment(s) described may include a particular feature, structure, or characteristic, but every embodiment may not necessarily include the particular feature, structure, or characteristic. Moreover, such phrases are not necessarily referring to the same embodiment. Further, when a particular feature, structure, or characteristic is described in connection with an embodiment, it would be within the knowledge of one skilled in the art to affect such feature, structure, or characteristic in connection with other embodiments, whether or not explicitly described, unless clearly stated to the contrary. That is, the various individual elements described below, even if not explicitly shown in a particular combination, are nevertheless contemplated as being combinable or arrangeable with each other to form other additional embodiments or to complement and/or enrich the described embodiment(s), as would be understood by one of ordinary skill in the art.

Throughout the disclosure, although embodiments of a filament cutting device, tether device, and/or tether delivery device may be described with specific reference to medical devices and systems and procedures within the digestive system, it should be appreciated that such medical devices and methods may be used in association with tissues of the abdominal cavity, gastrointestinal system, thoracic cavity, urinary and reproductive tract and the like. Moreover, a variety of medical procedures may benefit from the presently disclosed medical devices and procedures, including, for example, Endoscopic Submucosal Dissection (ESD), Peroral Endoscopic Myotomy (POEM), cholecystectomy and Video-Assisted Thorascopic Surgery (VATS) procedures. The structures and configurations, and methods of deploying, in order to stabilize, manipulate and provide a clear field of view may find utility beyond dissection.

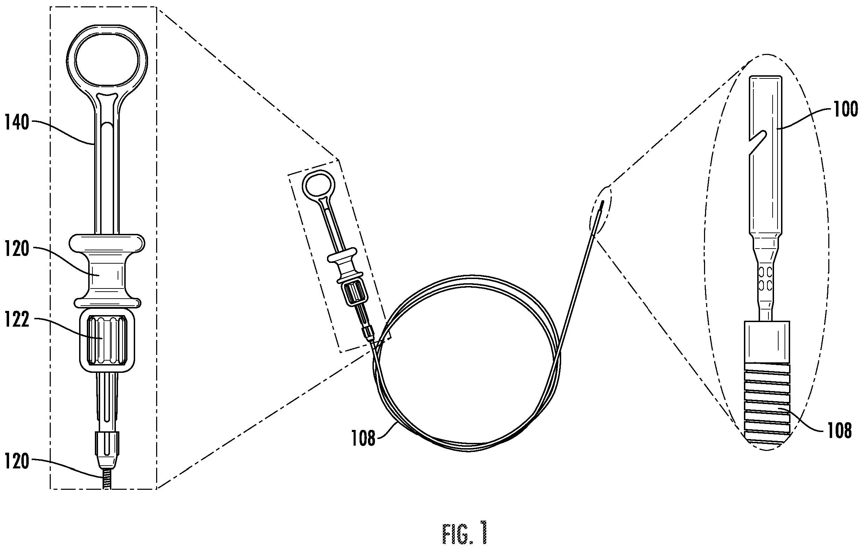

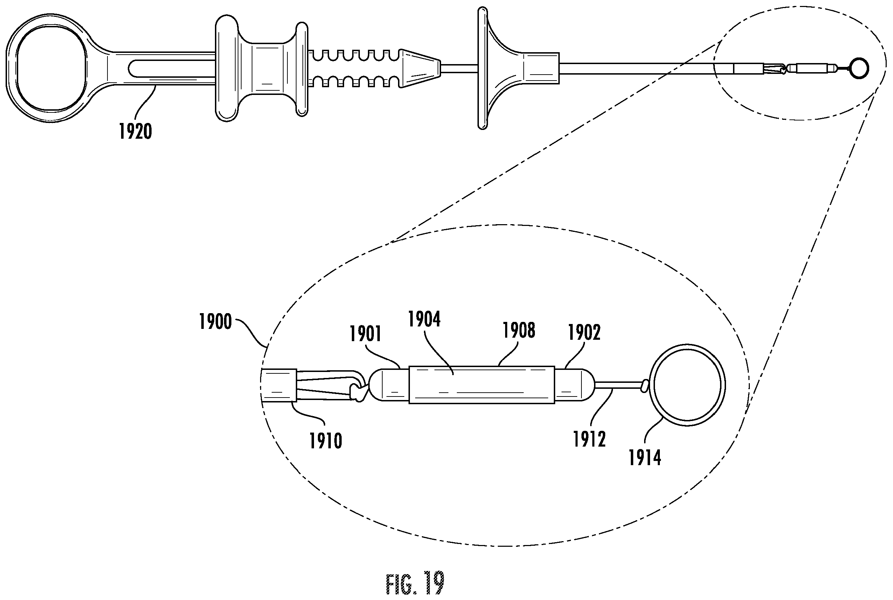

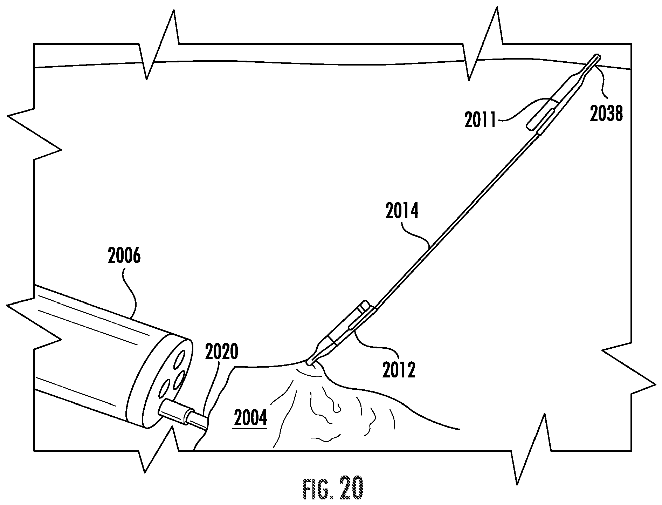

Referring to FIG. 1, an embodiment of a filament cutting device is depicted including an outer sheath 108 for extending within a body lumen of a patient. A filament may be a component connecting other elements together for performing a medical procedure (*see* FIGS. 19 and 20), or a filament may be a suture used for suturing or other closing of tissue. After performing the medical procedure, the filament may need to be severed to complete the procedure and remove components from the patient.

A distal end of the outer sheath 108 of the device is illustrated with an engaging body 100 extended distally out of the outer sheath 108. The engaging body 100 is extendable distally and proximally through the outer sheath 108. The outer sheath 108 may be formed as a coil, *e.g.,* to allow for increased bending motion in a tortuous anatomy. A proximal end of the device includes a handle 140 for manipulating the device and the engaging body 100 with respect to the outer sheath 108. The handle 140 is depicted with a finger slide 120 for translating the engaging body 100 along a longitudinal axis of the device relative to the outer sheath 108, and a rotating knob 122 for rotating the engaging body 100 about the longitudinal axis.

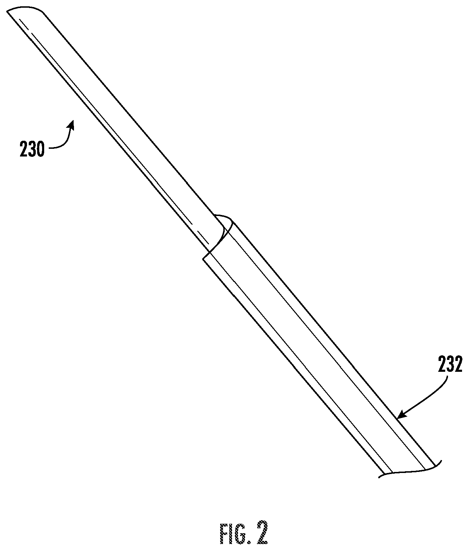

Referring to FIG. 2, an embodiment of an actuation element 230 is depicted within an inner sheath 232. The actuation element 230 may be connected to an engaging body such that the actuation element 230 and/or the inner sheath 232 may be translated proximally or distally or may be rotated about a longitudinal axis of a device to manipulate the engaging body relative to an outer sheath of the device. The actuation element 230 may be slidable with respect to the inner sheath 232 or they may axially extend together. The inner sheath 232 and the actuation element 230 may be disposed within an outer sheath of a device. The actuation element 230 may comprise a stiffer material (*e*.*g*., nitinol or the like) than that of the inner sheath such that the actuation element 230 may axially translate through the inner sheath 232 and/or the device. The inner sheath 232 may comprise a less stiff material (*e*.*g*., PTFE or the like) than the actuation element 230 such that contact of the stiffer actuation element 230 with the outer sheath is reduced. The actuation element 230 may be axially translated within the inner sheath 232 without substantial contact with the outer sheath, thereby reducing frictional forces between the substantially axial translation of the actuation element 230 and the outer sheath. The actuation element 230 and/or the inner sheath 232 may be coated, *e.g.,* with silicone or the like to reduce friction. An actuation element 230 may be, *e.g.,* a wire, a rod, or the like. An actuation element 230, such as an actuation wire, of any embodiment described herein or otherwise within the scope of the present disclosure may or may not include an inner sheath 232.

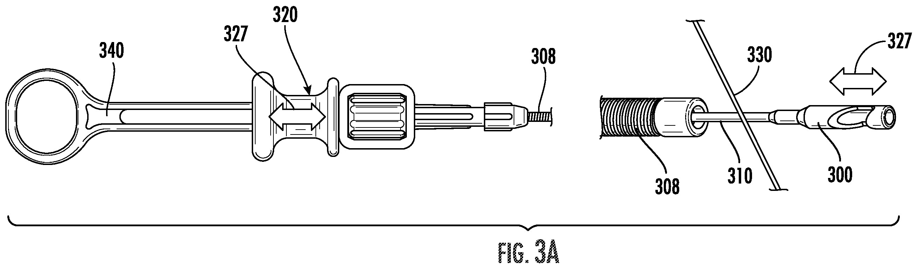

Referring to FIG. 3A, an embodiment of a filament cutting device is illustrated including an outer sheath 308 extending from a proximal end of the device to a distal end of the device. A slidable body 320 (*e.g.,* a finger slide) may be axially translated (*e.g.,* in the direction of the arrows 327) by a hand (*e.g.,* index and middle fingers of a hand) of a medical professional along a longitudinal axis of a handle 340 at the proximal end of the device to axially translate an actuation wire 310 and engaging body 300 with respect to the outer sheath 308 and a filament 330 to be cut. It is understood that in some embodiments, the handle 340 may translate axial movement of the outer sheath 308 relative to the actuation wire 310 and engaging body 300, such that the outer sheath 308 is retractable and advanceable while the actuation wire 310 and engaging body 300 remain stationary.

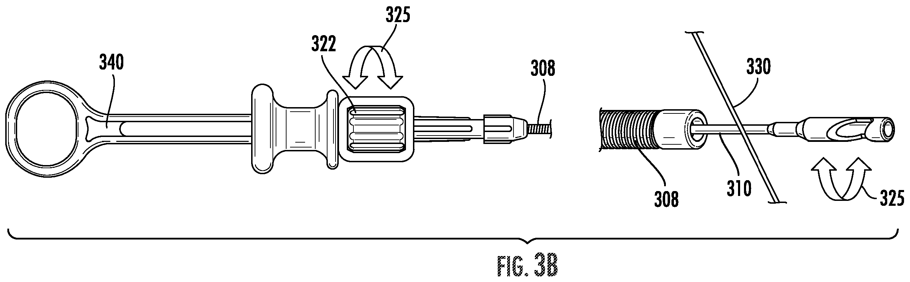

Referring to FIG. 3B, the filament cutting device of FIG. 3A is illustrated including a rotatable body 322 at the handle 340 that may be rotated about the longitudinal axis of the proximal end of the device (*e.g.,* in the direction of the arrows 325) by a hand (*e.g.,* thumb and finger of a hand) of a medical professional to rotate the actuation wire 310 and engaging body 300 with respect to the outer sheath 308 and a filament 330 to be cut.

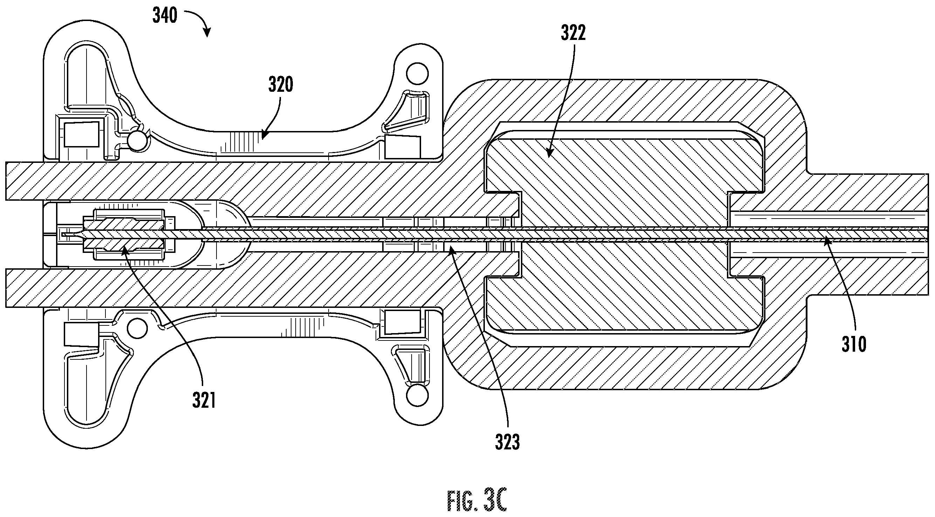

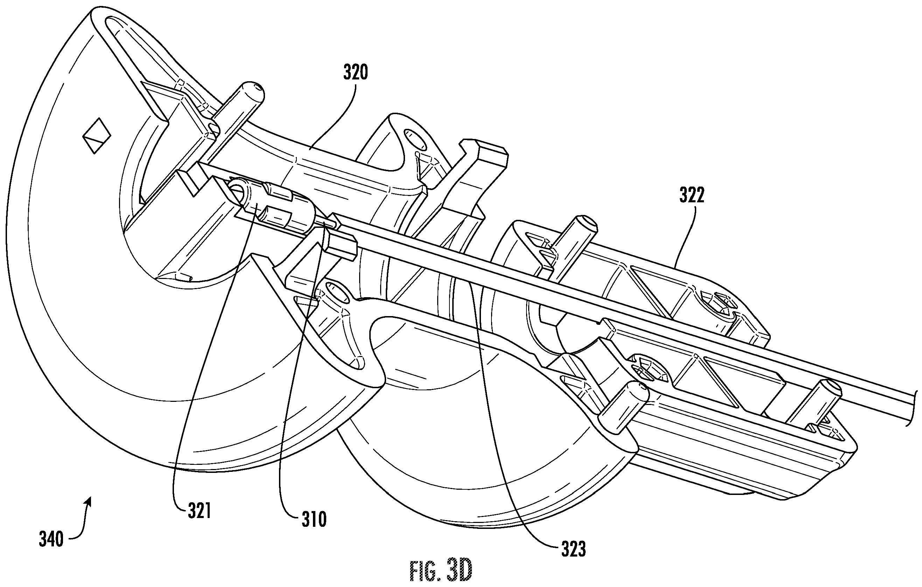

Referring to FIGS. 3C and 3D, a cross-sectional view of the handle 340 proximal end of the filament cutting device of FIGS. 3A and 3B is illustrated with the actuation wire 310 extending through the proximal end of the device. A proximal end of the actuation wire 310 is coupled to a shaft 321 that is rotatably coupled to the slidable body 320. An axial translation of the slidable body 320 axially translates the shaft 321 and the actuation wire 310. The actuation wire 310 is coupled to a tubular member 323. The tubular member 323 extends through the rotatable member 322 such that rotation of the rotatable member 322 translates rotation to the tubular member 323 and the actuation wire 310. In various embodiments, the tubular member 323 may be a cannula, a hypotube, or the like, and may have a cross-sectional shape that is round, oblong, square, rectangular, a combination thereof, or the like for translating rotation between the rotatable member 322 and the actuation wire 310. The tubular member 323 may extend solely within the proximal end of the filament cutting device (*e.g.,* only extending within the handle 340), or the tubular member 323 may also extend distally along the actuation wire 310 to a distal end of the filament cutting device. In various embodiments, the actuation wire 310 may include an inner sheath (*e.g.,* the inner sheath 232 of FIG. 2). The inner sheath may also be coupled to the shaft 321. In various embodiments, the tubular member may be an inner sheath (*e.g.,* the inner sheath 232 of FIG. 2).

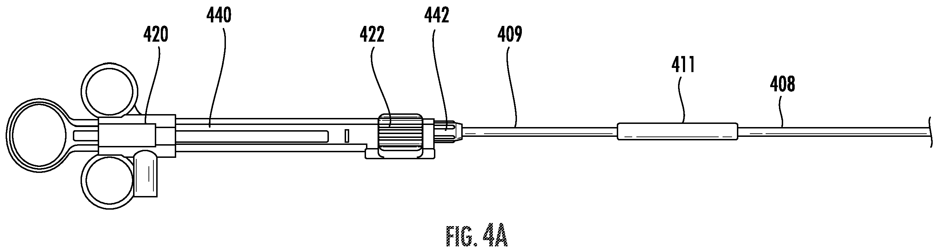

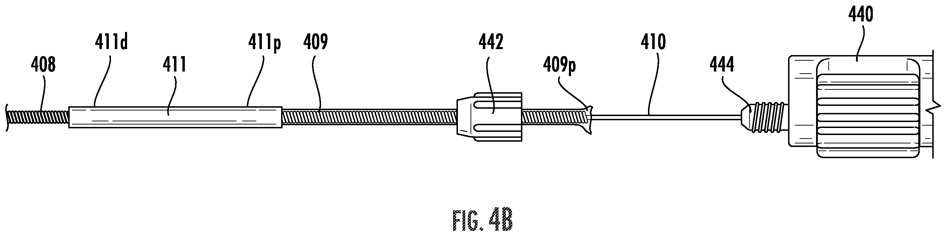

Referring to FIG. 4A, a proximal portion of an assembled filament cutting device according to an embodiment of the present disclosure is illustrated including a handle 440. The handle 440 includes a slidable body 420 for manipulating the device axially and a rotatable body 422 for manipulating the device rotationally. The mechanics for axial and rotational movement may be configured identical to or substantially similar to that described above with respect to the embodiments of FIGS. 1-3D. A proximal portion of an outer sheath 408 includes a strain relief tube 409 that is coupled to an outer surface of the outer sheath 408 by a heat shrink tube 411. A strain relief tube 409 may comprise of a material such as polypropylene or the like. FIG. 4B illustrates the unassembled device of FIG. 4A, revealing a proximal end 409p of the strain relief tube 409 including a flare having larger outer and inner diameters than a remainder of the tube 409. The proximal end 409p of the tube 409 is coupled to the handle 440 by securing the proximal end 409p to a threaded protrusion 444. An actuation wire 410 extends from the handle 440, through the threaded protrusion 444, and through the outer sheath 408. The flare of the proximal end 409p may be placed over and/or adjacent the protrusion 444 along the actuation wire 410 to assemble the device. The strain relief tube 409 is coupled to the handle 440 by a cap 442 mating with the threaded protrusion 444 such that the flare of the proximal end 409p of the tube 409 is held (*e.g.,* compressed, constrained, *etc.*) between the cap 442 and the threaded protrusion 444. The heat shrink tube 411 when melted couples the strain relief tube 409 to the outer sheath such that a proximal end 411p of the heat shrink tube bonds to the strain relief tube 409 and a distal end 411d of the heat shrink tube 411 bonds to the outer sheath 408.

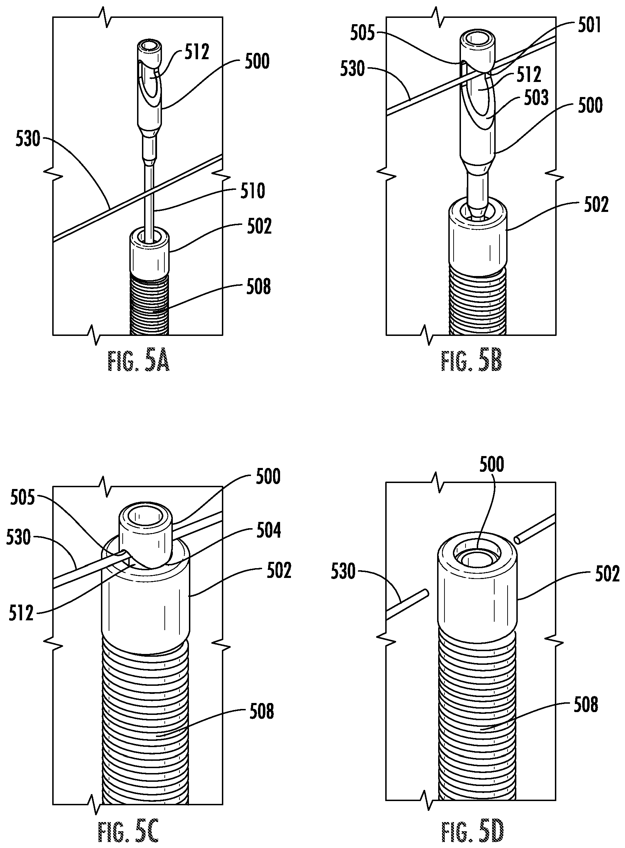

Referring to FIG. 5A, a distal end of an embodiment of a filament cutting device is illustrated including an engaging body 500 distally extendable out of a bushing 502 coupled to a distal end of an outer sheath 508. The engaging body 500 is distally extendable relative to a filament 530 for severing via translation of an actuation wire 510 coupled to the engaging body 500 relative to the outer sheath 508.

In various embodiments, an outer diameter of an engaging body may substantially match an inner diameter of a bushing. A bushing may be a cylindrical tube for receiving an engaging member that may be substantially cylindrical. One or both of an engaging body and a bushing may be substantially straight along a longitudinal axis of the engaging body and/or the bushing. An engaging body and/or bushing may be constructed so that one or both are more rigid than an actuation wire for the engaging body (*e.g.,* a wider or thicker engaging body than an actuation wire) and/or the shaft of the outer member that is connected to the bushing. The more rigid construction of the engaging body and/or bushing may provide the strength to sever a filament working in combination, while the less rigid actuation wire and outer member shaft for the bushing may provide flexibility to navigate tortious anatomies and may allow for bending along pathways of anatomy. The relative rigidity and width of the engaging body compared to the bushing may also improve pushability of engaging body through the outer member. A lubricious fluid or coating may be applied to one or both of the bushing and/or the engaging body such that they are slidable with respect to each other. In various embodiments, an engaging body or a bushing may comprise stainless steel, 304 stainless steel, nitinol, a polymer, or the like.

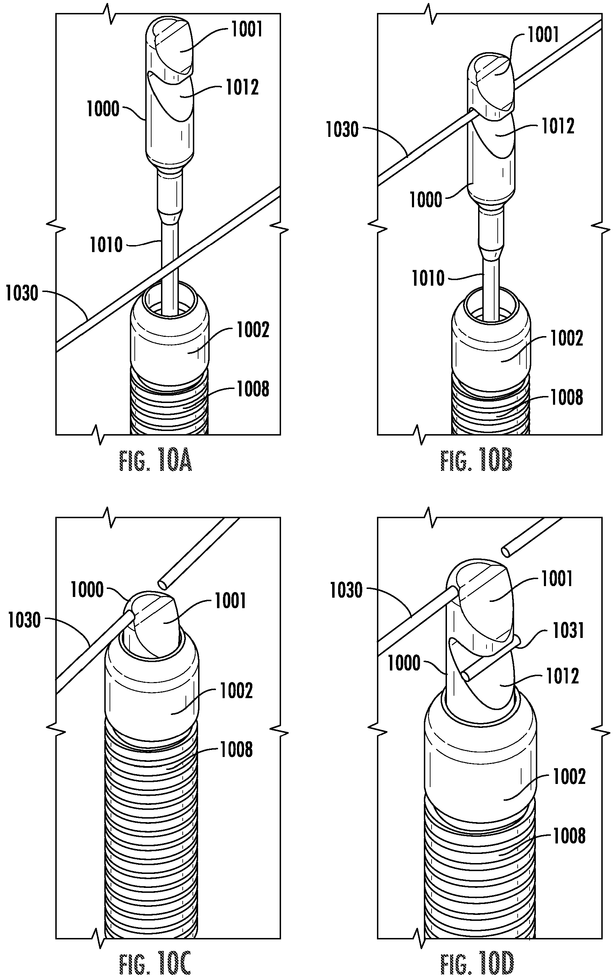

Referring to FIG. 5B, the engaging body 500 of FIG. 5A is proximally translated relative to the bushing 502 and the filament 530. The filament 530 is captured within a substantially radial cavity 512 of the engaging body 500. The filament 530 may be captured by proximal translation of the engaging body 500 with the filament 530 sliding along an outer surface of the engaging body 500. The filament 530 may slide along an angled sloping surface 503 of a proximal portion of the engaging body 500 that defines a perimeter of the cavity 512. The angled sloping surface 503 may be a lead-in for the filament 530 until contacting an innermost curvature of the cavity 512 defining a hook portion 505. The hook portion 505 may be formed so that the filament 530 is substantially perpendicular across the bushing 502 when the engaging body 500 is aligned with the bushing 502. For example, the innermost curvature of the cavity 512 may be defined on two sides of the engaging body 500. Each side may be positioned relative to each other such that the filament 530 may be perpendicular relative to the engaging body 500 and/or bushing 502. That is, one side of the filament may not be positioned at an angle relative to the engaging body 500 or bushing 502 different from another side of the filament across the engaging body 500 or bushing 502. The filament 530 may be prevented from moving distally past the cavity 512 by contacting a distal portion 501 of the engaging body 500 that extends proximally at the perimeter of the cavity 512. Although the cavity 512 is depicted as an aperture including a lumen of the engaging body 500, in various embodiments the engaging body 500 may be solid and the radial cavity 512 may instead be defined by a solid engaging body 400 (*e.g.,* as illustrated in FIGS. 10A-10D). In various embodiments, the angled sloping surface 503 may be uniform with the remainder of the perimeter of the cavity 512, may wider at the surface 503 and uniform along the remainder of the perimeter of the cavity 512, or may be wider at the angled sloping surface 503 and taper along the perimeter of the cavity 512. A wider surface 503 and/or a tapering perimeter of the cavity 512 may assist with capturing and positioning of the filament 530 within the cavity 512.

Referring to FIG. 5C, the engaging body 500 of FIGS. 5A and 5B may be translated proximally with respect to the outer sheath 508 and within the bushing 502 of the outer sheath 508 with the filament 530 captured within the cavity 512. The bushing 502 includes a lumen defined at a distal end of the bushing 502 by a substantially sharp edge 504 (*e.g.,* compared to an atraumatic blunt outer edge or surface of the distal end of the bushing 502). The engaging body 500 is substantially straight along a longitudinal axis of the engaging body 500 and is aligned with the bushing 502. An outer diameter of the engaging body 500 may substantially match, or be a slip fit to, an inner diameter of the bushing 502 at the edge 504 such that as the filament 530 is proximally translated to the edge 504 by the cavity 512 of the engaging body 500, a shear force between the edge 504 and the outer surface of the engaging body 500, or an edge defining a distal portion of the cavity 512, severs the filament 530. In various embodiments, the edge defining the cavity 512 (*e.g.,* the hook portion 505 of the cavity) may be substantially sharp and/or the edge 504 of the bushing 502 may be substantially sharp. In various embodiments, the bushing 502 and outer sheath 508 may be distally translatable with respect to the engaging body 500 and the filament 530.

Referring to FIG. 5D, the engaging body 500 of FIGS. 5A-5C may be further proximally translated within the bushing 502 and the outer sheath 508 such that a severed portion (not illustrated) of the filament 530 is captured within the bushing 502 and/or the outer sheath 508 for removal. It is also understood that the filament 530 may be severed at a single point by the bushing 502 and/or the cavity 512 such that no additional portion of the filament 530 is captured within the bushing 502 and/or the outer sheath 508. The device may be removed from the patient with the cut filament 530 left temporarily (*e*.*g*., graspers or other end effectors may secure and remove the cut filament 530) or permanently within the patient.

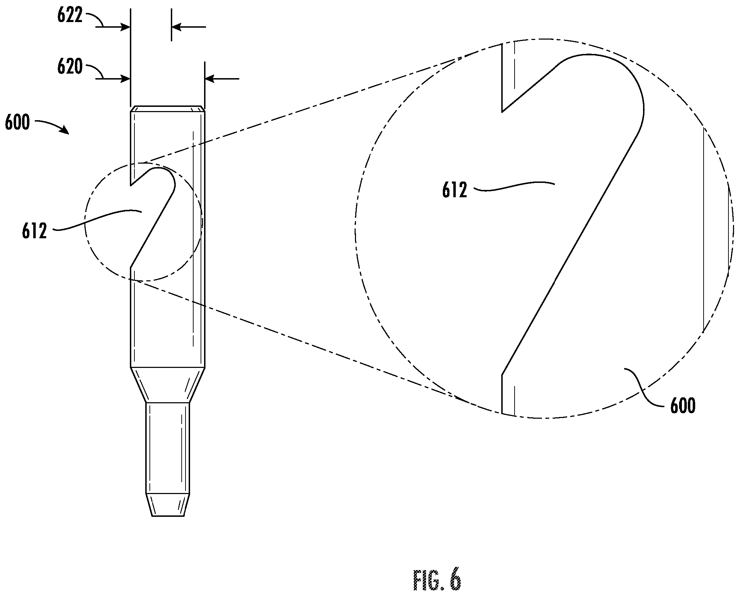

With reference to FIG. 6, a side view profile (and accompanying detail view) of a cavity 612 of an embodiment of an engaging body 600 is illustrated. The engaging body 600 includes a decreasing outer diameter in a proximal direction along the engaging body 600 to a proximal portion of the engaging body 600 that may couple to an actuation wire. The diameter may be tapered to guide in the engaging body 600 relative to the bushing and/or an outer sheath without having to be in exact alignment (*e*.*g*., axially along a longitudinal axis of either/both of the engaging body 600 and the bushing). In some embodiments, the engaging body 600 may be a constant diameter along its length. The cavity 612 may have a depth 622 from an outer surface of the engaging body 600 that is larger than 50% of an outer diameter 620 of the engaging body 600. In various embodiments, the depth 622 may have a length that is about 50% of the outer diameter 620. The profile of the cavity 612 may have numerous shapes including a hook-like shape, which may aid in retaining a filament when contacting the innermost surface of the cavity 612. Exemplary dimensions of the profile of the cavity 612 may include, *e.g.,* that a lead-in angled surface of a proximal portion of the cavity 612 includes an angle of about 30° from an outer surface of the engaging body 600. A lead-out angled surface distal to the lead-in angled proximal surface of the cavity 612 may include an angle of about 50° from an outer surface of the engaging body 600. Angles herein can vary and be chosen as desired depending on a given application, *e.g.,* a lead-in angled proximal surface and/or a lead-out angled surface of the cavity 612 may be about 0° to about 90° from the outer surface of the engaging body 600. It will be appreciated that other dimensions are contemplated and within the scope of the present disclosure.

In various embodiments, an engaging body and/or a bushing may include a cavity as described herein. A filament may be captured by axial translation of one or both of an engaging body or a bushing. A filament may slide along an outer surface of an engaging body and/or a bushing. A filament may slide along an angled sloping surface of a proximal or distal portion of an engaging body or a bushing that defines a perimeter of the cavity. A filament may be prevented from moving out of and/or past the cavity by contacting a perimeter of the cavity that extends back towards an opposing end of the cavity (*e*.*g*., forming a hook-like shape).

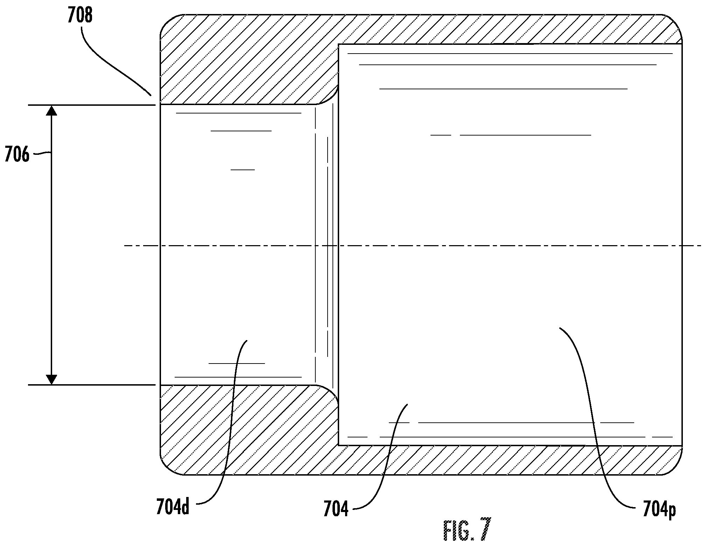

With reference to FIG. 7, a cross-sectional view of an embodiment of a bushing is illustrated including a lumen 704 therethrough. The lumen 704 has a proximal portion 704p having a wider diameter than a distal portion 704d such that the proximal portion 704p of the lumen 704 may be disposed about a distal end of an outer sheath. The distal portion 704d of the lumen 704 has a diameter 706 that may substantially match an outer diameter of an engaging body such that an internal edge 708 of a distal end of the bushing may create a shear force with the engaging body sufficient to sever a filament. The bushing may be fixedly coupled to the distal end of the outer sheath, *e.g.,* by welding, soldering, brazing, adhesive, gluing, mechanical fasteners, and the like. In some embodiments, the outer sheath and the bushing may be integrally formed, and in other embodiments, the outer sheath and the bushing may be joined together.

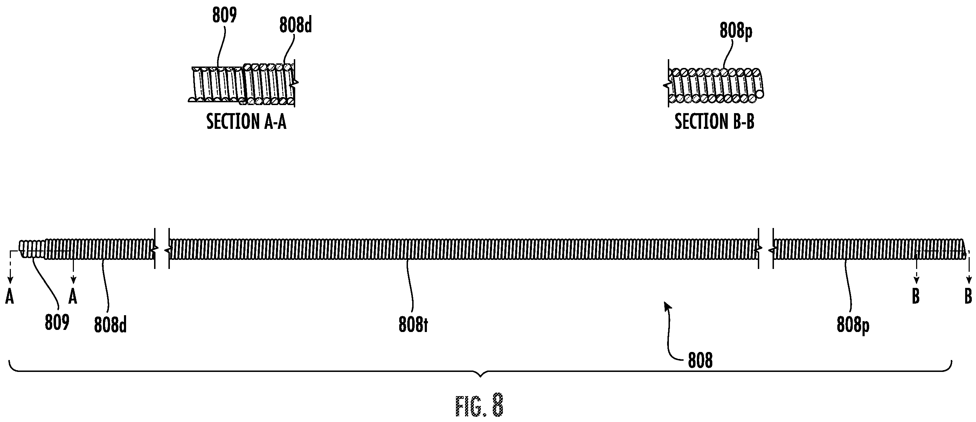

Referring to FIG. 8, an embodiment of an outer sheath 808 is illustrated (with accompanying cross-sectional views respectively along lines **A-A** and **B-B**) comprising a coiled body. The coiled body may be formed with a variable outer diameter over one or more mandrels. An outer sheath 808 having a coiled body may allow for more radial flexibility and axial stiffness than a solid uniform-walled outer sheath. A proximal portion 808p of the outer sheath 808 has a smaller inner diameter and a smaller outer diameter than the remainder of the outer sheath 808, which may assist with maintaining a lower profile and maneuverability compared to a remainder of the device. The proximal portion 808p of the outer sheath 808 extends distally to a tapered section 808t of the outer sheath 808. The tapered section 808t includes a distally increasing outer diameter and a distally increasing inner diameter. The tapered section 808t extends to a distal portion 808d of the outer sheath 808 that is depicted with a substantially uniform outer diameter. The distal portion 808d may be treated by grinding (*e.g.,* longitudinally grinding, polishing, or the like) to form the substantially uniform outer diameter. The diameter of the distal portion 808d of the outer sheath 808 may substantially match or be a slip fit within an inner diameter of a working channel (*e.g.,* about 2.8 mm or the like) of an endoscope. A distal tip 809 of the outer sheath 808 extending from the distal portion 808d may be ground, *e.g.,* as illustrated in FIG. 8, more than the distal portion 808d is ground, such that an outer diameter of the distal tip 809 is smaller than the outer diameter of the distal portion 808d. The outer diameter of the distal tip 809 may substantially match an inner diameter of a proximal portion of a lumen of a bushing such that the bushing may be disposed over and attachable to the distal tip 809, and the outer diameter of the bushing may substantially match the outer diameter of the distal portion 808d of the outer sheath 808. The inner diameter of the distal tip 809 may match the inner diameter of the distal portion 808d of the outer sheath 808 and an inner diameter of a distal portion of the bushing such that an engaging body may slidably translate within the distal portion 808d and distal tip 809 of the outer sheath 808 and through the bushing. In various embodiments, an outer sheath 808 may have a substantially uniform outer diameter along its length.

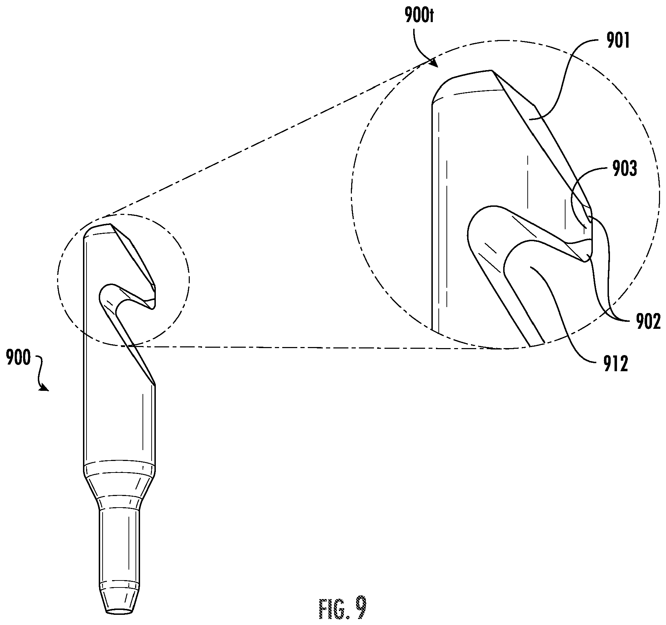

With reference to FIG. 9, an engaging body 900 is illustrated including a transverse distal tip 900t surface transitioning to a sloped surface 901 extending proximally at an angle along a longitudinal axis of the engaging body 900. The sloped surface 901 is substantially distal to a cavity 912 of the engaging body 900 along the length of the engaging body 900. The cavity 912 is illustrated as being formed within or defined by a solid body of the engaging body 900, however the cavity 912 may include a substantially radial aperture formed within the engaging body 900. The sloped surface 901 transitions to an outer circumferential surface 903 of the engaging body 900 in a proximal direction and continues extending to the cavity 912. The sloped surface 901 may assist with capturing a filament within the cavity 912 of the engaging body as illustrated and discussed with respect to a sloped surface of FIGS. 10A-10D. The transitions between the sloped surface 901 and the outer circumferential surface 903, and the outer circumferential surface 903 to the cavity 912, may each include fillets 902 (*e.g.,* rounded surfaces, smoothed surfaces, atraumatic surfaces, or the like). The fillets 902 may reduce friction with other portions of a device, another device, and/or a patient anatomy compared to non-filleted edges. During a procedure, axial viewing of an engaging body 900, portions of an engaging body 900, and/or the orientation of an engaging body 900 or a cavity 912 may be difficult for a medical professional to identify. The fillets 902 may provide surfaces that are identifiable to the medical professional compared to other surfaces of the engaging body 900 (*e.g.,* by reflecting light at a different angle or reflecting light in a different shape than other surfaces of the engaging body 900).

Referring to FIGS. 10A-10D, a distal end of an embodiment of a filament cutting device is illustrated substantially similar to that discussed with respect to FIGS. 5A-5D. In FIGS. 10A-10D, a distal portion of an engaging body 1000 includes a sloped surface 1001. The sloped surface 1001 may slide along a filament 1030, for example, as an actuation wire 1010 and engaging body 1000 are translated distally into contact with the filament 1030. During a distally-traveling contact of the engaging body 1000 with the filament 1030, the filament 1030 may slide along the sloped surface 1001 proximally toward a cavity 1012 of the engaging body 1000. Contacting a distal tip of an engaging body 1000 including the sloped surface 1001 with the filament 1030 may be easier to facilitate capture of the filament 1030 within the cavity 1012 compared to a distal tip of an engaging body 1000 that has a substantially transverse surface that may collide with the filament 1030 and direct the filament 1030 away from the cavity 1012. The engaging body 1000 may include filleted surfaces along the outside of the engaging body from the distal tip to the cavity 1012 to help prevent the filament 1030 from being damaged or severed prematurely. The filament 1030 may be captured within the cavity 1012 and proximally retracted for severing within a bushing 1002 of an outer sheath 1008 by proximal translation of the engaging body 1000 relative to the outer sheath 1008. The engaging body 1000 may remain stationary after capturing the filament 1030, and the bushing 1002 and outer sheath 1008 may extend distally to sever the filament 1030 as the engaging body 1000 is received into the outer sheath 1008. The engaging body 1000 may be translated distally with respect to the bushing 1002 to eject a severed portion 1031 of the filament 1030.

In various embodiments, a filament may be severed in a variety of ways. For example, a filament may be severed by a cut, a plastic break, a tensioned break, or the like that may be performed by a cutter that is mechanical, electrical, chemical, or the like. In various embodiments, a filament of a device may be cut during or at the termination of a medical procedure. Severing a filament may be performed for a variety of reasons including, *e.g.,* to release a device, to remove a filament such as a suture, to release tension between devices, between a device and an anatomy, between anatomies, between a first portion of an anatomy and a second portion of an anatomy, or the like.

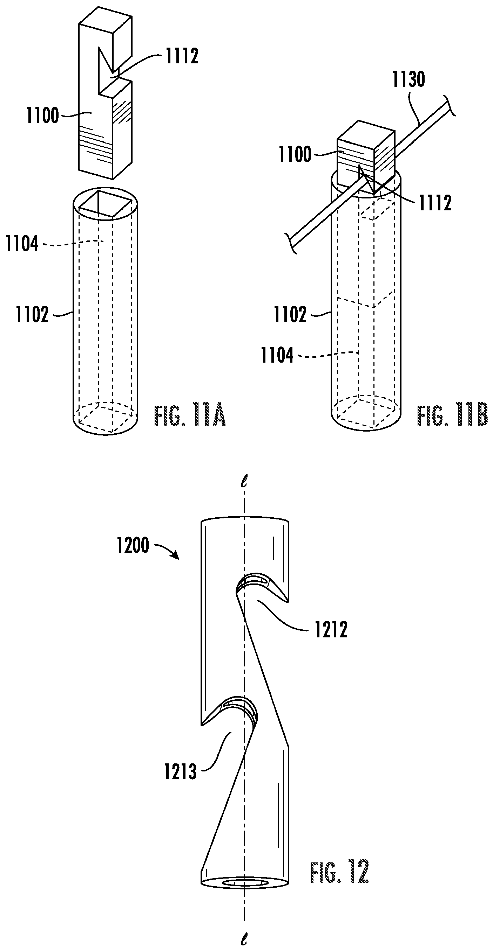

Referring to FIGS. 11A and 11B, an embodiment of an engaging body 1100 and a bushing 1102 are illustrated. The engaging body 1100 may be coupled to an actuation wire and the bushing 1102 may be coupled to an outer sheath as described herein. The engaging body 1100 is slidable within a lumen 1104 of the bushing 1102. The engaging body 1100 has a non-circular perimeter (*e*.*g*., square, rectangular, polyhedral, or the like) that substantially matches the non-circular lumen 1104. A cavity 1112 of the engaging body 1100 may be used to capture a filament 1130 to sever the filament 1130 between the cavity 1112 and the bushing 1102 via translation of the engaging body 1100 toward the bushing 1102 and/or translation of the bushing 1102 toward the engaging body 1100. In embodiments, such as the one illustrated, the perimeter of the engaging body 1100 and the cavity 1112 do not include any curved surfaces. In some embodiments, non-circular perimeter geometries of the engaging body 1100 may be advantageous in that fewer inputs, less time, and/or tolerance controls may be utilized than others for manufacturing.

Referring to FIG. 12, an embodiment of an engaging body 1200 is illustrated including a first cavity 1212 and a second cavity 1213. The cavities 1212, 1213 are oriented substantially opposite each other about a longitudinal axis ℓ of the engaging body 1200. The second cavity 1213 allows for another portion of the engaging body 1200 to capture a filament. The substantially opposite orientation of the cavities 1212, 1213 allows for minimal rotation of the engaging body 1200 in order to capture a filament (*i.e.,* a smaller rotation of the engagement member 1200 about the longitudinal axis ℓ may be required to expose a cavity 1212, 1213 to a filament compared to a larger rotation that may be required with an embodiment having only a first cavity 1212). Both cavities 1212, 1213 are arranged such that they have a depth extending through the longitudinal axis ℓ (*i.e.,* beyond 50% of the outer diameter of the engaging body 1200); however, in various embodiments, the cavities may extend up to or radially short of the longitudinal axis ℓ. For example, the lead-in proximal angle of the cavities 1212, 1213 with respect to an outer surface of the engaging body 1200 may be smaller such that the cavities 1212, 1213 do not radially extend past the longitudinal axis ℓ. The cavities 1212, 1213 overlap with each other along the longitudinal axis ℓ and also radially overlap transversely through the longitudinal axis ℓ. However, the cavities 1212, 1213 may be arranged such that they do not overlap with each other along the longitudinal axis ℓ, do not radially overlap transversely through the longitudinal axis ℓ, and/or may not be arranged substantially opposite each other about the longitudinal axis ℓ. Although two cavities 1212, 1213 are illustrated, any number of cavities may be employed, *e.g., 0,* 1, 3, 4, 5, 8, 10, 20, *etc.* Although the cavities 1212, 1213 are illustrated as arranged approximately 180° about the longitudinal axis ℓ, any angled arrangement may be employed, *e.g.,* about 60°, about 90°, about 120°, about 150°, *etc.*

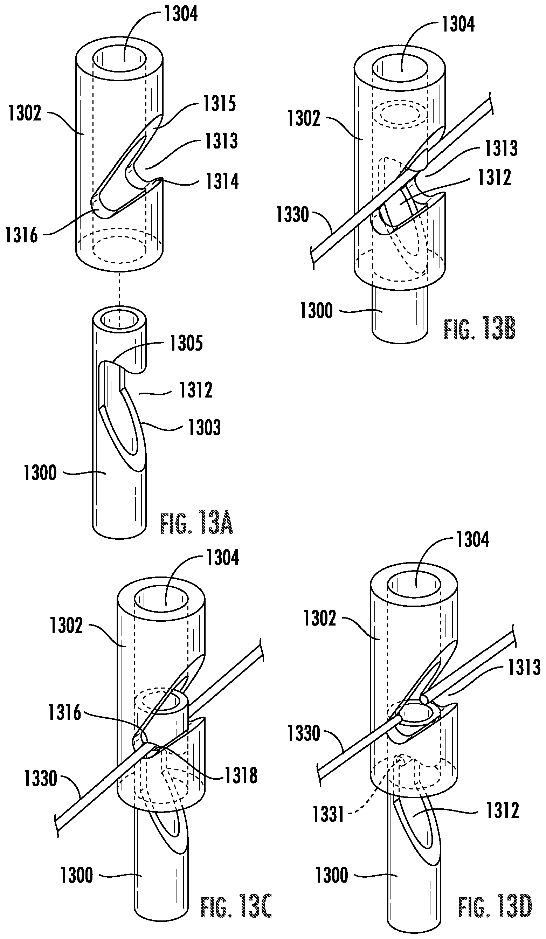

With reference to FIG. 13A, an embodiment of an engaging body 1300 and a bushing 1302 are illustrated. The engaging body 1300 may be coupled to an actuation wire and the bushing 1302 may be coupled to an outer sheath as described herein. The engaging body 1300 is slidable within a lumen 1304 of the bushing 1302. The engaging body 1300 includes a substantially radial first cavity 1312 and an angled sloping surface 1303 of a proximal portion of the engaging body 1300 that defines a perimeter of the first cavity 1312. The angled sloping surface 1303 of the first cavity 1312 extends to an innermost curvature of the first cavity 1312 defining a hook portion at a second surface 1305 of the first cavity 1312. Although the first cavity 1312 is depicted as an aperture including a lumen of the engaging body 1300, in various embodiments the engaging body 1300 may be solid and the radial cavity 1312 may instead be defined by a solid engaging body 1300 (*e.g.,* as illustrated in FIGS. 10A-10D). The bushing 1302 includes a substantially radial second cavity 1313 that extends into the lumen 1304. The second cavity 1313 includes a proximal surface 1314 and a distal surface 1315 that meet at a curved midportion 1316 of the second cavity 1313. The proximal surface 1314 and the distal surface 1315 are angled such that the outer portions of the surfaces 1314, 1315 are farther apart from each other compared to the inner portions of the surfaces 1314, 1315 (*i.e.,* towards the midportion 1316). This orientation of the proximal and distal surfaces 1314, 1315 of the second cavity 1313 forms a perimeter of the second cavity 1313 such that the second cavity 1313 has a wider outer portion at the outer surface of the engaging body 1300 and a narrower inner portion at the curved midportion 1316.

Referring to FIG. 13B, the first and second cavities 1312, 1313 of FIG. 13A may be substantially aligned to accept and capture a filament 1330. The bushing 1302 may be distally extended such that the filament 1330 enters the widest outer portion of the second cavity 1313. The filament 1330 may be moved into the second cavity 1313 along the angled perimeter of the second cavity 1313. The second cavity may direct the filament 1330 proximally toward the narrowest inner portion of the second cavity 1313. At the inner portion of the second cavity 1313 the filament 1330 is proximal to the distal surface (*i.e.,* the distal surface 1315 of FIG. 13A) of the first cavity 1312 when the cavities 1312, 1313 are aligned.

Referring to FIG. 13C, with the filament 1330 captured within the first and second cavities 1312, 1313 of FIGS. 13A and 13B, the engaging body 1300 may be translated proximally with respect to the bushing 1302. As the first and second cavities 1312, 1313 move past each other, the filament 1330 is subjected to shearing forces between a distal perimeter outer edge 1316 of the first cavity 1312 and a proximal inner edge 1318 of the perimeter of the second cavity 1313.

Referring to FIG. 13D, as the first and second cavities 1312, 1313 of FIGS. 13A-13C move past each other across the filament 1330, the filament 1330 is severed. A severed portion 1331 of the filament 1330 may be kept within the lumen 1304, withdrawn proximally through the lumen 1304, expelled distally through the lumen 1304, or expelled substantially radially out of the second cavity 1313.

In various embodiments, a cavity of an engaging body and/or a bushing may include various shapes, surfaces, and/or edges for engaging, accepting, trapping, moving, sliding, stopping, guiding, shearing, and/or or holding a filament or a portion of a filament. A combination of various portions of shapes and/or surfaces of cavities depicted and described with respect to a particular embodiment or embodiments may be used across other embodiments of cavities described or otherwise within the scope of the present disclosure.

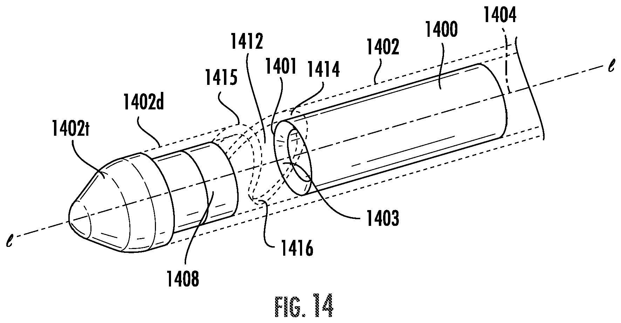

Referring to FIG. 14, an embodiment of an engaging body 1400 and a bushing 1402 are illustrated. The engaging body 1400 may be coupled to an actuation wire and the bushing 1402 may be coupled to an outer sheath as described herein. The engaging body 1400 is slidable within a lumen 1404 of the bushing 1402. The bushing 1402 includes a substantially radial cavity 1412 that extends into the lumen 1404. The cavity 1412 includes a proximal surface 1414 and a distal surface 1415 that meet at a curved midportion 1416 of the cavity 1412. The engaging body 1400 includes a cutting edge 1401 at a distal tip of the engaging body 1400 at an outer diameter of the engaging body 1400. The distal tip of the engaging body 1400 includes a surface 1403 having an angle extending from a longitudinal axis ℓ of the engaging body 1400 to the cutting edge 1401. The surface 1403 extends both proximally and inwardly from the cutting edge 1401 towards the longitudinal axis ℓ. A filament may be captured within the cavity 1412 and the engaging body 1400 may be translated within the lumen 1404 toward the filament within the cavity 1412. A contact body 1408 is disposed within a distal end 1402d of the bushing 1402 that prevents distal translation of the engaging body 1400. The contact body 1408 may comprise a material that is soft enough such that the cutting edge 1401 is not damaged when it contacts the contact body 1408 and resilient enough such that the cutting edge 1401 may extend at least partially into the contact body 1408 distally past a filament to ensure a complete severing a filament, for example, comprising a material such as urethane, high density polyethylene, a plasticized grade of PVC, or the like. The bushing 1402 includes an atraumatic tip 1402t with a distally narrowing diameter such that anatomies or other instruments may not be harmed while delivering the device and/or such that narrow pathways may be easier to traverse compared to a device without the atraumatic tip 1402t.

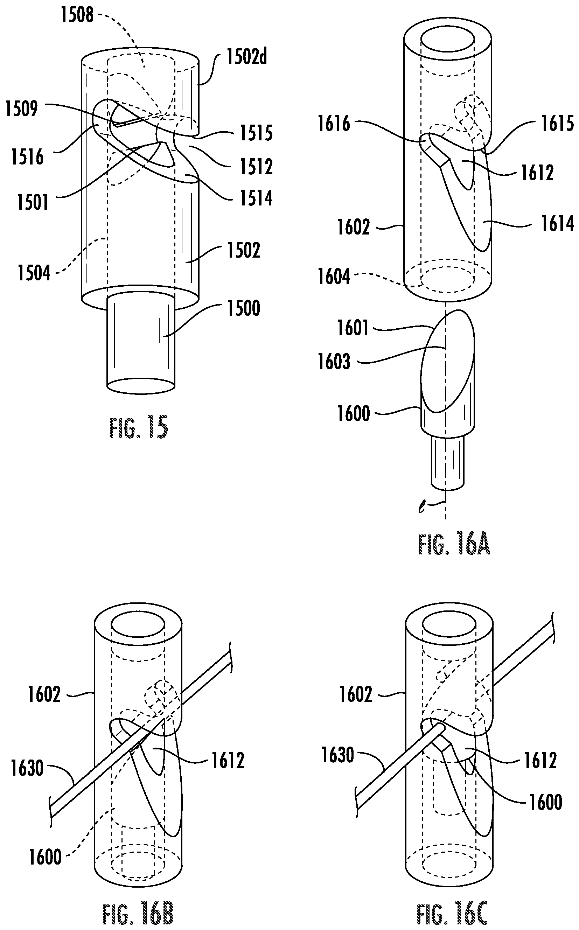

With reference to FIG. 15, an embodiment of an engaging body 1500 and a bushing 1502 are illustrated. The engaging body 1500 may be coupled to an actuation wire and the bushing 1502 may be coupled to an outer sheath as described herein. The engaging body 1500 is slidable within a lumen 1504 of the bushing 1502. The bushing 1502 includes a substantially radial cavity 1512 that extends into the lumen 1504. The cavity 1512 includes a proximal surface 1514 and a distal surface 1515 that meet at a curved midportion 1516 of the cavity 1512. The engaging body 1500 includes a cutting portion 1501 at a distal tip of the engaging body 1500 that may be a cutting edge or a blunt surface. The cutting portion 1501 is a distal portion of the engaging body 1500 that tapers distally with a decreasing width. A filament may be captured within the cavity 1512 and the engaging body 1500 may be translated within the lumen 1504 toward the filament within the cavity 1512. A contact body 1508 is disposed within a distal end 1502d of the bushing 1502 that prevents distal translation of the engaging body 1500. The contact body 1508 comprises a tapered proximal portion 1509 that tapers proximally with a decreasing width. With a filament captured within the cavity 1512, the engaging body 1500 may be translated distally towards the contact body 1508 such that the filament is compressed between the cutting portion 1501 and the proximal portion 1509 and/or sheared between the cutting portion 1501 and the proximal portion 1509, thereby severing the filament.

Referring to FIGS. 16A-16C, an embodiment of an engaging body 1600 and a bushing 1602 are illustrated. The engaging body 1600 may be coupled to an actuation wire and the bushing 1602 may be coupled to an outer sheath as described herein. The engaging body 1600 is slidable within a lumen 1604 of the bushing 1602. The bushing 1602 includes a substantially radial cavity 1612 that extends into the lumen 1604. The cavity 1612 includes a proximal surface 1614 and a distal surface 1615 that meet at a curved midportion 1616 of the cavity 1612. The engaging body 1600 includes a cutting edge 1601 at a distal tip of the engaging body 1600 at an outer diameter of the engaging body 1600. The distal tip of the engaging body 1600 includes a surface 1603 having an angle extending across a longitudinal axis ℓ of the engaging body 1600 to the cutting edge 1601. The angled surface 1603 may be used to trap a filament and the cutting edge 1601 about the angled surface 1603 may decrease a required amount of shear stress to sever a filament compared to a radial cross-sectional surface with a shorter perimeter about the surface. A filament 1630 may be captured within the cavity 1612 and the engaging body 1600 may be translated within the lumen 1604 toward the filament 1630 within the cavity 1612. As the cutting edge 1601 is translated distally past the cavity 1612, the cutting edge 1601 and an inner edge of the distal surface 1615 shear the filament 1630.

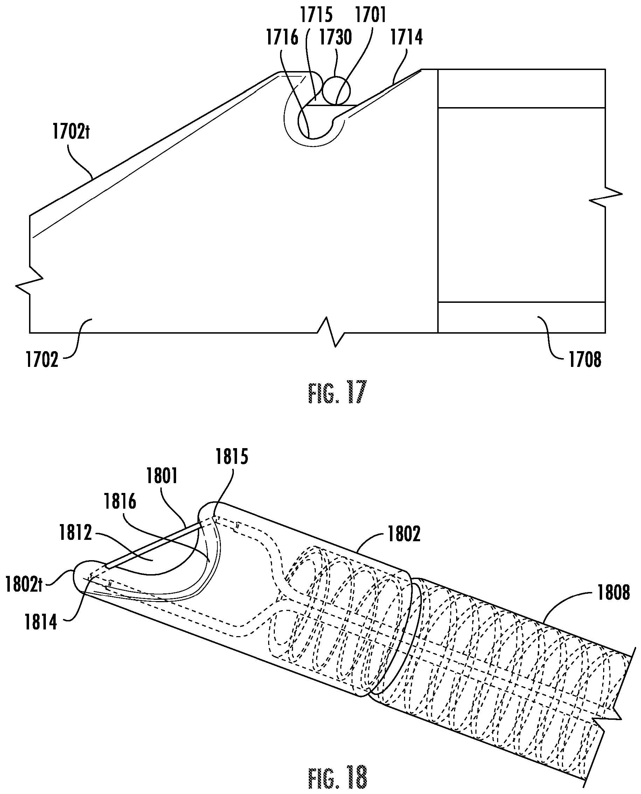

Referring to FIG. 17, an embodiment of a filament cutting device is illustrated including a bushing 1702 coupled to a distal end of the outer sheath 1708. The bushing 1702 includes a substantially radial cavity 1712. The cavity 1712 includes a proximal surface 1714 and a distal surface 1715 that meet at a curved midportion 1716 of the cavity 1712. A cutter 1701 extends across the cavity 1712. The cutter 1701 extends substantially parallel with a longitudinal axis of the bushing 1702, but may be angled, *e.g.,* parallel or normal to the proximal surface 1714. The cutter 1701 includes an edge oriented substantially radially outward from the cavity 1712. A filament 1730 may be captured within the cavity 1712 and the bushing 1702 may be translated proximally and/or radially against the filament 1730 such that the cutter 1701 severs the filament 1730. A distance between an outer surface of the bushing 1702 to the edge of the cutter within the cavity 1712 may be substantially equal to a diameter of a filament 1730, *e.g.,* about 0.25 millimeters or the like, such that the distal surface 1715 may be placed adjacent the filament 1730 for manipulation of the filament 1730 and/or to act as a backstop against the filament 1730 for cutting. The embodiment of FIG. 17 has no moving parts with respect to each other, which may reduce stress on the outer sheath 1708 and/or the filament 1730 during operation.

Referring to FIG. 18, an embodiment of a filament cutting device is illustrated including a bushing 1802 coupled to a distal end of an outer sheath 1808. The bushing 1802 includes a cavity 1812. The cavity 1812 is defined substantially transversely across a distal tip 1802t of the bushing 1802 and includes a curved midportion 1816 of the cavity 1812. A cutter 1801 extends across the cavity 1812. The cutter 1801 extends substantially transversely across a longitudinal axis of the bushing 1802, but may be angled. The cutter 1801 is an activatable wire configured to melt a filament. Ends 1814, 1815 of the cutter 1801 extend into the bushing 1802 and are coupled (*e.g.,* welded) to a first lead wire 1851 and a second lead wire 1852 that extend proximally along the bushing 1802 and the outer sheath 1808 to an energy source (*e.g.,* a battery within a handle). The cutter 1801 and leads 1851, 1852 may be overmolded within the bushing 1802. The cutter 1801 has a conductive outer surface while the leads 1851, 1852 are insulated by the bushing 1802 and/or insulative coverings along the leads 1851, 1852. The embodiment of FIG. 18 has no moving parts, which may reduce stress on the outer sheath 1808 and/or a filament during operation. The cutter 1801 may comprise various conductive materials such as nichrome, iron-chromium-aluminum alloy, or the like.

Referring to FIG. 19, an embodiment of a tether device 1900 is illustrated including an elastic, stretchable body 1904 having first 1901 and second ends 1902. An elongate tubular hollow body alignment member 1908 is extendable at least partially over the elastic body 1904. The alignment member 1908 may align and/or orient the device 1900 within a working channel of a scope, other introducer sheath, or catheter during device 1900 manipulation. A clip 1910 is coupled to the first end 1901 of the elastic body 1904. A neck 1912 extends from the second end 1902 of the elastic body 1904 to a loop 1914. The clip 1910 may be manipulated by a medical professional such that the clip 1910, coupled to the first end 1901 of the tether device 1900, is delivered toward a tissue. The clip 1910 may be coupled to the tissue in addition to being coupled to the first end 1901 of the elastic body 1904. The loop 1914 may be engaged by another device such as an additional clip. The additional clip may be moved to position the loop 1914 within the additional clip jaws and to couple the additional clip to another anatomy or another portion of the tissue such that the second end 1902 of the elastic member 1904 extends away from the first end 1901. In this position, the tether device 1900 is placed in greater axial tension compared to a relaxed state of the tether device 1900 that is illustrated in FIG. 19. In various embodiments, a clip 1910 may be rotatable to rotate the tether device 1900. A clip 1910 may be repositionable before, during, and/or after a procedure. A clip 1910 may be a single use clip. With the tether device 1900 and the tissue(s) coupled to the tether device 1900 in tension, a medical procedure may be performed, *e*.*g*., resecting of the tissue. During and/or after the procedure, tension may be released by severing a filament of the tether device such as the elastic body 1904, the alignment member 1908, the neck 1912, and/or the loop 1914 (see *e.g.,* FIGS. 45-5D and 10A-10D). In various embodiments, the elastic body 1904 may be severable by the cutting device. In various embodiments, an elastic body 1904 may include one or more securing bodies at one or more ends 1901, 1902 of the elastic body 1904 that may each be coupled to a filament. An elastic body 1904 may include an internal filament that may prevent the elastic body 1904 from stretching beyond a desirable length. A filament of an elastic body 1904 may comprise, extend to, or be coupled to one or more loops (*e.g.,* loop 1914 with or without a neck 1912) that can be various shapes and diameters.

Referring to FIG. 20, an embodiment of a tether device is illustrated as delivered and applying tension between a target tissue 2004 and another tissue 2038. An elastic body 2014 is coupled to a first clip 2012 at a first end of the elastic body 2014. The first clip 2012 is coupled to the target tissue 2004 for resection. A second end of the elastic body 2014 is coupled to a second clip 2011. The second clip 2011 is coupled to tissue 2038 such that the elastic body 2014 is in tension. A resecting tool 2020 is delivered toward the target tissue 2004 via an endoscope 2006. As the target tissue 2004 is resected, the elastic body 2014 pulls the first clip 2012 and the target tissue 2004 substantially toward the second clip 2011 such that visualization between the endoscope 2006, the tool 2020, and the target tissue 2004 is maintained. During or at the termination of the procedure, an embodiment of a filament cutting device may be delivered to the elastic body 2014 to cut the elastic body 2014, releasing tension in the elastic body 2014. Various embodiments of a tether device and clip delivery device, or other delivery device for a tether device, such as the tether device and clip delivery device of FIG. 19, may be used in a tissue dissection procedure, such as the procedure depicted in FIG. 20.

An embodiment of a method of cutting a filament may include inserting a device having an outer sheath into a patient. An engaging body and/or a bushing of the device may be manipulated toward the filament. The filament may be captured in one or more cavities of the device. The engaging body and/or a bushing may be translated axially along the device thereby cutting the filament.

All of the devices and/or methods disclosed and claimed herein can be made and executed without undue experimentation in light of the present disclosure. While the devices and methods of this disclosure have been described in terms of preferred embodiments, it may be apparent to those of skill in the art that variations can be applied to the devices and/or methods and in the steps or in the sequence of steps of the method described herein without departing from the concept, spirit and scope of the disclosure. All such similar substitutes and modifications apparent to those skilled in the art are deemed to be within the spirit, scope and concept of the disclosure as defined by the appended claims.

### The following aspects are preferred embodiments of the invention.

1. A filament cutting device, comprising:
   an outer sheath;
   a bushing coupled to a distal end of the outer sheath, wherein an inner diameter of the bushing includes a cutting edge;
   an actuation wire slidably extendable within the outer sheath and bushing; and
   an engaging body coupled to a distal end of the actuation wire, the engaging body comprising a cavity defined along a length of the engaging body configured to capture a portion of a filament within the cavity; and
   wherein movement of the actuation wire and engaging body with the filament captured within the cavity causes the cutting edge to sever the filament.
2. The filament cutting device of aspect 1, wherein a proximal portion of the cavity comprises an angled sloping surface and a distal portion of the cavity comprises an innermost curvature of the cavity defining a hook shape.
3. The filament cutting device of aspect 2, wherein the innermost curvature of the cavity extends radially within the engaging body a length that is greater than 50% of a diameter of the engaging body.
4. The filament cutting device of any of aspects 1-3, wherein the cutting edge is at a distal tip of the bushing.
5. The filament cutting device of any of aspects 1-4, further comprising a cutting cavity defined along a length of the bushing, and wherein the cutting edge is along the cutting cavity.
6. The filament cutting device of any of aspects 1-5, wherein the outer sheath comprises winding coils, and a distal tip of the outer sheath comprises a ground outer surface where the bushing is coupled to the outer sheath.
7. The filament cutting device of any of aspects 1-6, wherein a proximal portion of the outer sheath comprises a smaller inner diameter than a remainder of the outer sheath, and wherein the proximal portion of the outer sheath comprises a smaller outer diameter than the remainder of the outer sheath.
8. The filament cutting device of any of aspects 1-7, wherein the engaging body further comprises a second cavity substantially opposing the first cavity about a longitudinal axis of the engaging body.
9. The filament cutting device of any of aspects 1-8, wherein the engaging body comprises a substantially square outer perimeter that substantially matches an inner perimeter of the bushing.
10. A filament cutting device, comprising:
   an outer sheath;
   a bushing coupled to a distal end of the outer sheath, a cavity defined along a length of the bushing configured to capture a portion of a filament within the cavity;
   an actuation wire slidably extendable within the outer sheath and the bushing;
   an engaging body coupled to a distal end of the actuation wire, the engaging body comprising a cutting edge at a distal tip of the engaging body; and
   wherein movement of the actuation wire and engaging body with the filament captured within the cavity causes the cutting edge to sever the filament.
11. The filament cutting device of aspect 10, wherein the cutting edge is an outer diameter of the engaging body.
12. The filament cutting device of aspect 11, wherein a distal tip of the engaging body comprises a surface having an angle extending from a longitudinal axis of the engaging body to the cutting edge.
13. The filament cutting device of any of aspects 10-12, further comprising a contact body disposed within a distal end of the bushing configured to prevent distal translation of the engaging body.
14. The filament cutting device of any of aspects 10-13, wherein the contact body comprises a tapered proximal portion that tapers proximally with a decreasing width.
15. The filament cutting device of any of aspects 10-14, wherein the engaging body comprises a tapered distal portion that tapers distally with a decreasing width.

## Claims

1. A filament cutting device, comprising:
an outer sheath (1008);
a bushing (1002) coupled to a distal end of the outer sheath, wherein the bushing includes a cutting edge;
an actuation wire (1010) slidably extendable within the outer sheath and bushing; and
an engaging body (900, 1000) coupled to a distal end of the actuation wire, the engaging body comprising a cavity (912, 1012) defined along a length of the engaging body configured to capture a portion of a filament (1030) within the cavity, the engaging body including at its distal portion a sloped surface (901, 1001) slidable along the filament (1030) as the engaging body (1000) are translated distally into contact with the filament (1030); and
wherein movement of the actuation wire and engaging body with the filament captured within the cavity causes the cutting edge to sever the filament.

2. The filament cutting device of claim 1, wherein an inner diameter of the bushing includes the cutting edge.

3. The filament cutting device of claim 1 or 2, wherein the sloped surface extends proximally at an angle along a longitudinal axis of the engaging body.

4. The filament cutting device of any preceding claim, wherein the sloped surface is configured to assist with capturing a filament within the cavity.

5. The filament cutting device of any preceding claim, wherein the engaging body (1000) includes filleted surfaces along an outside of the engaging body from its distal tip to the cavity (1012) to help prevent the filament from being damaged or severed prematurely.

6. The filament cutting device of any preceding claim, wherein a proximal portion of the cavity comprises an angled sloping surface (503, 1303) and a distal portion of the cavity comprises an innermost curvature of the cavity defining a hook shape.

7. The filament cutting device of claim 6, wherein the innermost curvature of the cavity extends radially within the engaging body a length that is greater than 50% of a diameter of the engaging body.

8. The filament cutting device of any preceding claim, wherein the cutting edge is at a distal tip of the bushing.

9. The filament cutting device of any preceding claim, further comprising a cutting cavity (1313) defined along a length of the bushing, and wherein the cutting edge is along the cutting cavity.

10. The filament cutting device of any preceding claim, wherein the outer sheath comprises winding coils, and a distal tip of the outer sheath comprises a ground outer surface where the bushing is coupled to the outer sheath.

11. The filament cutting device of any preceding claim, wherein a proximal portion of the outer sheath comprises a smaller inner diameter than a remainder of the outer sheath, and wherein the proximal portion of the outer sheath comprises a smaller outer diameter than the remainder of the outer sheath.

12. The filament cutting device of any preceding claim, wherein the engaging body further comprises a second cavity (1213) substantially opposing the first cavity (1212) about a longitudinal axis of the engaging body.

13. The filament cutting device of any preceding claim, wherein the engaging body (1100) comprises a substantially square outer perimeter that substantially matches an inner perimeter of the bushing.
